# EUROPEAN PATENT SPECIFICATION

(11) **EP 2 062 582 B1**
(45) Date of publication and mention of the grant of the patent: **17.04.2013**
(21) Application number: 07800696.2
(22) Date of filing: 14.08.2007
(51) Int. Cl.: A61K 31/424, A61K 31/43, A61K 31/431, A61K 31/545, A61K 31/546, A61K 31/7036, A61K 45/06, A61K 9/00, A61K 9/19, A61K 47/02, A61K 47/12, A61K 47/18, A61K 47/26, A61P 31/04, A61P 31/00

(54) **ANTIBIOTIC COMPOSITION COMPRISING BETA-LACTAM ANTIBIOTICS, AMINOGLYCOSIDES AND BUFFERS**
ANTIBIOTISCHE ZUSAMMENSETZUNG MIT BETA-LACTAM-ANTIBIOTIKA, AMINOGLYKOSIDEN UND PUFFERN
COMPOSITIONS ANTIBIOTIQUES COMPRENANT DES BÊTALACTAMINES, DES AMINOGLYCOSIDES ET DES TAMPONS

(30) Priority: 25.08.2006 CN 200610015438
(43) Date of publication of application: 27.05.2009
(60) Divisional application: 12171437.2
(73) Proprietor: Tianjin Hemay Bio-Tech Co., Ltd., Tianjin 300457 (CN)
(72) Inventor: ZHANG, Hesheng, Tianjin, 300457 (CN)
(74) Representative: Hryszkiewicz, Danuta
(86) International application number: PCT/CN2007/002441
(87) International publication number: WO 2008/025228

(56) References cited:
- EP-A1- 1 468 697
- WO-A1-2006/085337
- CN-A- 1 485 035
- CN-A- 1 565 457
- CN-A- 1 593 423
- CN-A- 1 660 116
- GB-A- 759 951
- IN 1241DEL2005 A1 (VENUS REMEDIES LTD) 18 Agust 2006 (2006-08-18) -& Database WPI Week 200711 Thomson Scientific, London, GB; AN 2007-110449 XP002542735

## Description

Field of the Invention

The invention relates to a pharmaceutical composition comprising at least one β-lactam antibiotic, at least one aminoglycoside and at least one buffer component. The pharmaceutical composition optionally further comprises at least one β-lactamase inhibitor;. The pharmaceutical composition is useful as an anti-microbial drug.

Background of the invention

β-Lactam antibiotics inhibit the synthesis of bacterial cell wall by inhibiting the activity of D-alanyl-D-alanine transpeptidase (peptidoglycan transpeptidase) inside bacteria. Peptidoglycans are linear polysaccharide polypeptides with a network structure alternately comprising N-acetyl-glucosamine (Glc-NAc) and Mur-NAc. The transpeptide cross-linking reaction of these linear polymers catalysed by peptidoglycan transpeptidase results in network architecture and completes cell wall synthesis. β-Lactam antibiotics irreversibly inhibit the activity of the peptidoglycan transpeptidase and cause a failure of bacterial cell wall formation. Without cell wall, bacterial cells don't have a definite shape and sustain high permeation pressure inside cells, which causes bacteriolysis resulting in the death of bacteria. β-Lactam antibiotics are the most widely used antibiotics in clinical medicine.

Bacteria have subsequently evolved to produce β-lactamase, which can hydrolyze the amido bond of the β-lactam ring of β-lactam antibiotics and transform β-lactam antibiotics into metabolites lacking antibacterial activity. In 1976, it was discovered that clavulanic acid separated from the fermented fluid of rod-like streptomycete was a unique β-lactamase inhibitor. Soon thereafter, other β-lactamase inhibitors, especially sulbactam and its lipid prodrugs, i.e., composition of ampicillin sodium and sulbactam sodium, and tazobactam became widely used in clinical settings.

Another type of antibiotics widely used is aminoglycoside antibiotics. Aminoglycoside antibiotics are glycosides formed from aminosugar (monosaccharide or disaccharide) and amino-cyclitol. They are alkaline in nature owing to their amino and other basic functional groups. Due to their broad anti-bacterial spectrum, high anti-bacterial activity, frequent clinical use, there have been more than 20 species of aminoglycosides developed since the discovery of the first aminoglycoside antibiotic, streptomycin, which was isolated from Streptothrix in 1940.

The anti-bacterial mechanism of action of aminoglycoside antibiotics is entirely different from that of β-lactam antibiotics. After entering bacteria the aminoglycoside antibiotic conjugates with the 30S subunit protein, which causes errors when tRNA translates mRNA code and results in non-functioning proteins inhibiting cell growth.

It is generally known that the combination of β-lactam antibiotics with aminoglycoside antibiotics provides an anti-bacterial synergy. However, β-lactam antibiotics are acidic whereas aminoglycoside antibiotics are basic. When these two types of antibiotics are dissolved in the same solution, either a salt precipitates out due to acid-base reaction, or the amino group of the aminoglycoside antibiotic reacts with the β-lactam group of the β-lactam antibiotics. Both of the reactions drastically reduce the efficacy of these types of antibiotics. Therefore, mixing of these two types of antibiotics in the same solution is normally disadvantageous.

EP 1 468 697 discloses pharmaceutical compositions containing piperacillin (as piperacillin sodium) and tazobactam (as tazobactam sodium), further including an aminocarboxylic acid chelating agent, for example, ethylene diamine tetraacetic acid (EDTA), or a pharmaceutically acceptable salt thereof, optionally a buffer, most preferably a citrate, and optionally an aminoglycoside (tobramycin or amikacin). EP 1 468 697 discloses preparing liquid samples by thawing a frozen bag comprising piperacillin sodium, tazobactam sodium, dextrose hydrous, and sodium citrate dehydrate, reconstituting with dextrose 5%, filtering, optionally adding EDTA, and optionally adding an aminoglycoside. The content of piperacillin and the number of particulates in the liquid samples is assessed at time zero and at 24 hours.

WO 2006/120705 which was filed on 8 May 2006 and was published on 16 Nov. 2006, which is after the priority date of the instant application and, therefore, should be treated as Art 54(3) prior art for novelty purposes only, discloses the following combinations of ceftazidime and tobramycin: (a) 1 g ceftazidime and 0.12 g of tobramycin, (b) 2 g of ceftazidime and 0.18 g tobramycin, and (c) 500 mg ceftazidime and 60 mg tobramycin. WO 2006/120705 further discloses that the combinations are stable for prolonged periods of time as dry powders at 40°C and 75% RH. WO 2006/120705 also discloses a method for making a liquid composition of tobramycin and ceftazidime by dissolving EDTA in water for injection, adding sodium meta bi sulfite to this solution with continuous stirring and nitrogen purging, adding a buffer of 0.017M sodium citrate and 0.01 M citric acid of pH 5.8, followed by addition of tobramycin and ceftazidime one by one with continuous stirring below 25 °C, followed by addition of phenol, addition of water, readjustment of pH and charcoal treatment during filtration with 0.2 micron to get a colorless solution. However, WO 2006/120705 does not disclose any compositions comprising a β-lactamase inhibitor, does not provide any stability data of the liquid composition of ceftazidime and tobramycin alone over time, and does not indicate that the liquid composition of ceftazidime and tobramycin remains clear for any period except for at the time of mixing.

WO 2006/085337 A1 discloses a pharmaceutical composition comprising ceftriaxone and vancomycin as powder for injection. WO 2006/085337 A1 further discloses that the composition is stable for prolonged periods of time as dry powders at 40°C and 75% RH. Although WO 2006/085337 A1 does not offer any experimental data to show that ceftriaxone and vancomycin are stable in solution and that such solution remains clear and without the formation of particulates for any period of time, WO 2006/085337 A1 does stipulate that incorporation of sodium bicarbonate, L-arginine, EDTA, a neutralizing agent, a buffer, or a chemical vector into the dry powder composition makes the combination allegedly stable after reconstitution.

The development of a pharmaceutical composition, in which β-lactam antibiotics and aminoglycoside antibiotics could not only stably coexist without loss of efficacy but could also act in synergy, would bring about a breakthrough in the field of infectious diseases. The key to the development of such a composition is the discovery of a system, in which a mixture of β-lactam antibiotics and aminoglycoside antibiotics would not form a precipitate, and wherein a reaction between the β-lactam group of β-lactam antibiotics with the amino group of aminoglycoside antibiotics can be completely inhibited.

As a result of systematic studies, it has been found that when the pH value is controlled in the range of between 3 and 9, a precipitate resulting from a salt forming reaction and the reaction between the β-lactam group of β-lactam antibiotics with the amino group of aminoglycoside antibiotics can be inhibited to a certain degree; when the pH value is controlled in the range of between 4 and 8, the precipitate and the reaction between the amino group and the β-lactam group can be inhibited to a significant degree; and when the pH value is controlled in the range of between 6 and 7.5, the precipitate and the reaction between the amino group and the β-lactam group can be completely inhibited.

Summary of the invention

In view of the above-described problems, it is one objective of the invention to provide a pharmaceutical composition which can be used as an anti-microbial and anti-infection drug.

In order to achieve the above objectives, in accordance with one embodiment of the invention, provided is a pharmaceutical composition, comprising: at least one β-lactam antibiotic, at least an aminoglycoside antibiotic and at least one buffer component. A liquid formulation of the pharmaceutical composition is clear and transparent without turbidity or precipitate, and the efficacy of the β-lactam antibiotic is maintained for at least 8 hours.

In another class of this embodiment, a β-lactamase inhibitor is added to the pharmaceutical composition, the resultant solution is clear and transparent without turbidity or precipitate, and the efficacy of the β-lactam antibiotic, β-lactamase inhibitor, and aminoglycoside antibiotic in the composition is maintained for at least 8 hours.

In another class of this embodiment, the β-lactam antibiotic is cefoperazone, ceftriaxone, mezlocillin, or a pharmaceutically acceptable salt or hydrate thereof. Particularly, the β-lactam antibiotic is cefoperazone, ceftriaxone, or mezlocillin.

In another class of this embodiment, the aminoglycoside antibiotic is amikacin, gentamicin, etimicin, or a pharmaceutically acceptable salt or hydrate thereof. Particularly, the aminoglycoside antibiotic is amikacin, gentamicin, or etimicin.

In another class of this embodiment, the β-lactamase inhibitor is clavulanic acid, sulbactam, tazobactam, a combimation composition of ampicillin sodium and sulbactam sodium, or a pharmaceutically acceptable salt or hydrate thereof. Particularly, the β-lactamase inhibitor is sulbactam, or tazobactam.

In another class of this embodiment, the buffer system is citric acid/citrate system or any other organic polyacid buffer system, phosphoric acid/phosphate system or any other inorganic acid buffer system, acetic acid/acetate system or any other organic monoacid system, arginine system or any other amino acid system, tris/HCl system, or any other pharmaceutically acceptable buffer system. Particularly, the buffer solution system is citric acid/citrate system, phosphoric acid/phosphate system, acetic acid/acetate system, arginine system, or carbonic acid/carbonate system. More particularly, the buffer system is citric acid/citrate system, phosphoric acid/phosphate system, or acetic acid/acetate system. The buffer component in the examples of this invention is sodium citrate.

In another class of this embodiment, the effective pH range of the buffer solution is between 5.5 and 7.5. More particularly, the effective pH range of the buffer solution is between 6.0 and 6.75.

In another class of this embodiment, a concentration range of the buffer solution is between 1 and 500 mM; particularly, between 5 and 100 mM; and more particularly between 10 and 60 mM.

The pharmaceutical compositions described herein can be divided in four classes according to their clinical applications, as follows:

1) In the first class is a pharmaceutical composition, comprising: at least one β-lactam antibiotic, at least one β-lactamase inhibitor, at least one aminoglycoside antibiotic, and a buffer component. This pharmaceutical composition, formulated as an injectable solution, is clear and transparent without turbidity or precipitate, and the efficacy of the β-lactam antibiotic and the aminoglycoside antibiotic in this composition is maintained. A representative unit-dose composition in this class, comprises: between 0.1 g and 5 g of a β-lactam antibiotic, between 0.1 g and 5 g of a β-lactamase inhibitor, between 0.01 g and 5 g of sodium citrate, and between 0.01 g and 5 g of an aminoglycoside antibiotic. The pharmaceutical composition can be prepared as a solution preparation with freeze preservation, or a lyophilized injectable powder, which is dissolved prior to use.

2) In the second class is a pharmaceutical composition, comprising: at least one β-lactam antibiotic, at least one buffer component, and at least one aminoglycoside antibiotic. The working pH range of the buffer component is between 6 and 7. This pharmaceutical composition, formulated as an injectable solution, is clear and transparent without turbidity or precipitate, and the efficacy of the β-lactam antibiotic and the aminoglycoside antibiotic in this composition is maintained. A representative unit-dose composition in this class, comprises: between 0.1 g and 5 g of β-lactam antibiotic, between 0.01 g and 5 g of sodium citrate, and between 0.01 g and 5 g of an aminoglycoside antibiotic. The pharmaceutical composition can be prepared as a solution preparation stored with freeze preservation, or a lyophilized injectable powder, which is dissolved prior to use.

3) In the third class is a pharmaceutical composition, comprising: at least one β-lactam antibiotic, at least one β-lactamase inhibitor, and a buffer component. The working pH range of the buffer component is between 6 and 7. This pharmaceutical composition, formulated as an injectable solution, is clear and transparent without turbidity or precipitate. The efficacy of the β-lactam antibiotic and the aminoglycoside antibiotic in this composition is maintained when the two antibiotics are mixed together in a container. A representative unit-dose composition in this class, comprises: between 0.1 g and 5 g of β-lactam antibiotic, between 0.1 g and 5 g of β-lactamase inhibitor, and between 0.01 g and 5 g of sodium citrate. The pharmaceutical composition can be prepared as a solution preparation stored with freeze preservation, or a lyophilized injectable powder, which is dissolved prior to use.

4) In the fourth class is a pharmaceutical composition, comprising: at least one β-lactam antibiotic and at least one buffer component. When the pharmaceutical composition, formulated as an injectable solution, is mixed in a container with at least a solution of an aminoglycoside antibiotic, a clear and transparent mixture without turbidity or precipitate is obtained. The efficacy of the β-lactam antibiotic and the aminoglycoside antibiotic is maintained. A representative unit-dose composition in this class, comprises: between 0.1 g and 5 g of a β-lactam antibiotic, and between 0.01 g and 5 g of sodium citrate. The pharmaceutical composition can be prepared as a solution preparation stored with freeze preservation, or a lyophilized injectable powder, which is dissolved prior to use.

In clinical application of the pharmaceutical composition of the invention, salts or hydrates of β-lactam antibiotics are preferable.

In clinical application of the pharmaceutical composition of the invention, if β-lactamase inhibitor is needed, a salt of the β-lactamase inhibitor, such as potassium clavulanate, sulbactam sodium, tazobactam sodium or a hydrate thereof are preferable.

In clinical application of the pharmaceutical composition of the invention, the weight ratio of a β-lactam antibiotic to a β-lactamase inhibitor is preferably 1:1, 2:1, 4:1, or 8:1.

The pharmaceutical compositions described herein include, but are not limited, to the following representative unit dose Formulations:

Formulation 1: cefoperazone sodium 0.1-4 g, sulbactam sodium 0.1-4 g, amikacin sulfate 20-800 mg, sodium citrate 10-5000 mg, and citric acid 20-5000 mg.

Formulation 2: cefoperazone sodium 0.5-4 g, sulbactam sodium 0.1-4 g, 0.2-50 mg, gentamicin 20-800 mg, sodium citrate 10-5000 mg, and citric acid 20-1200 mg.

Formulation 3: cefoperazone sodium 0.5-4 g, sulbactam sodium 0.1-4 g, etimicin 20-800 mg, sodium citrate 10-5000 mg, and citric acid 20-1200 mg.

Formulation 4: cefoperazone sodium 0.5-4 g, tazobactam 0.1-4 g, amikacin sulfate 20-800 mg, sodium citrate 10-5000 mg, and citric acid 20-1200 mg.

Formulation 5: cefoperazone sodium 0.5-4 g, tazobactam 0.1-4 g, gentamicin 20-800 mg, sodium citrate 10-5000 mg, and citric acid 20-1200 mg.

Formulation 6: cefoperazone sodium 0.5-4 g, tazobactam 0.1-4 g, etimicin 20-800 mg, sodium citrate 10-5000 mg, and citric acid 20-1200 mg.

Formulation 7: cefoperazone sodium 0.5-4 g, potassium clavulanate 0.1-4 g, amikacin sulfate 20-800 mg, sodium citrate 10-5000 mg, and citric acid 20-1200 mg.

Formulation 8: cefoperazone sodium 0.5-4 g, potassium clavulanate 0.1-4 g, gentamicin 20-800 mg, sodium citrate 10-5000 mg, and citric acid 20-1200 mg.

Formulation 9: cefoperazone sodium 0.5-4 g, potassium clavulanate 0.1-4 g, etimicin 20-800 mg, sodium citrate 10-5000 mg, and citric acid 20-1200 mg.

Formulation 10: ceftriaxone sodium 0.5-4 g, sulbactam sodium 0.1-4 g, amikacin sulfate 20-800 mg, sodium citrate 10-5000 mg, and citric acid 20-1200 mg.

Formulation 11: ceftriaxone sodium 0.5-4 g, sulbactam sodium 0.1-4 g, gentamicin 20-800 mg, sodium citrate 10-5000 mg, and citric acid 20-1200 mg.

Formulation 12: ceftriaxone sodium 0.5-4 g, sulbactam sodium 0.1-4 g, etimicin 20-800 mg, sodium citrate 10-5000 mg, and citric acid 20-1200 mg.

Formulation13: cefoperazone sodium 0.5-4 g, sulbactam sodium 0.1-4 g, etimicin 20-800 mg, monosodium phosphate 0.1-2 g, disodium phosphate 0.5-4 g.

Formulation 14: cefoperazone sodium 0.5-4 g, tazobactam sodium 0.1-4 g, etimicin 20-800 mg, monosodium phosphate 0.1-2 g, disodium phosphate 0.5-4 g.

Formulation 15: cefoperazone sodium 0.5-4 g, sulbactam sodium 0.1-4 g, gentamicin 20-800 mg, monosodium phosphate 0.1-2 g, disodium phosphate 0.5-4 g.

Formulation 16: cefoperazone sodium 0.5-4 g, tazobactam sodium 0.1-4 g, gentamicin 20-800 mg, sodium acetate 10-5000 mg, acetic acid 100-1200 mg.

Formulation 17: cefoperazone sodium 0.5-4 g, sulbactam 0.1-4 g, gentamicin 20-800 mg, arginine 10-5000 mg.

Formulation 18: cefoperazone 0.5-4 g, tazobactam 0.1-4 g, gentamicin 20-800 mg, arginine 10-5000 mg.

Formulation 19: cefoperazone 0.5-4 g, sulbactam 0.1-4 g, gentamicin 20-800 mg, arginine 10-2000 mg.

Formulation 20: mezlocillin sodium 0.5-4 g, tazobactam sodium 0.05-5 g, amikacin sulfate 0.05-4 g, and sodium citrate 0.01-5 g.

Formulation 21: mezlocillin sodium 0.5-4 g, tazobactam sodium 0.05-5 g, gentamicin sulfate 0.05-4 g, and sodium citrate 0.01-5 g.

Formulation 22: mezlocillin sodium 0.5-4 g, tazobactam sodium 0.05-5 g, etimicin sulfate 0.05-4 g, and sodium citrate 0.01-5 g.

Formulation 23: mezlocillin sodium 0.5-4 g, sulbactam sodium 0.05-5 g, amikacin sulfate 0.05-4 g, and sodium citrate 0.01-5 g.

Formulation 24: mezlocillin sodium 0.5-4 g, sulbactam sodium 0.05-5 g, gentamicin sulfate 0.05-4 g, and sodium citrate 0.01-5 g.

Formulation 25: mezlocillin sodium 0.5-4 g, sulbactam sodium 0.05-5 g, etimicin sulfate 0.05-4 g, and sodium citrate 0.01-5 g.

An iso-osmotic solution may be used to prepare a parenteral solution of the pharmaceutical composition described in this invention. The iso-osmotic solution includes but is not limited to a glucose solution, a fructose solution, and normal saline. The unit dose range of glucose, fructose, or sodium chloride is between 0.1 g and 20 g; and the concentration range in the parenteral solution is between 0.1 % and 10% w/w.

Different formulations (vide supra) were prepared by selecting gentamicin, amikacin, or etimicin with one of four β-lactam antibiotic and one of two β-lactamase inhibitors.

Research on stability and intercomponent compatibility of three dosage forms and preservation methods was carried out. The results shows that in solution preparations prepared by the following three ways: 1) used immediately after preparation; 2) prepared and cryopreserved, then thawed, and then added an aminoglycoside antibiotic prior to use; and 3) prepared as solution, freeze-dried, and preserved at low temperature, then reconstituted as a solution, and then added an aminoglycoside antibiotic prior to use; the contents of the β-lactam antibiotic, β-lactamase inhibitor and aminoglycoside antibiotic were all maintained at levels higher than 90%, and on some cases higher than 95%, which meets technical requirements for clinically applied multi-drug mixtures.

The pharmaceutical composition provides patients and doctors a choice of simultaneous use of β-lactam antibiotics and aminoglycoside antibiotics, and a more effective treatment regimen. More importantly, due to the potential avoidance of drug resistance caused by use of single type of antibiotics, the pharmaceutical composition disclosed herein is more likely to prevent first-time-treatment failure.

The solution formulation of the pharmaceutical composition prepared for microbial control can be used as a parenteral solution, eye drops, nose drops, ear drops, genital duct drops, wash, or a solution for external use. The administration of the solution formulation of the pharmaceutical composition set forth herein as anti-microbial infection drug is preferably by intravenous injection.

The solution formulation of the pharmaceutical composition may be prepared immediately prior to use; or it may be prepared as solution formulation, then sealed, then preserved in a frozen state, and then thawed at room temperature prior to use.

The pharmaceutical composition may be prepared as a solution, injectable powder, or freeze-dried injectable powder and preserved with cold-storage, and re-dissolved into a liquid solution with injectable fluid immediately prior to use.

Still in other aspects of the invention, provided is a method for the preparation of a lyophilized powder of the pharmaceutical composition described herein, comprising: (a) dissolving a β-lactam antibiotic, and other components of the pharmaceutical composition (e.g., the components identified in Formulations 1 to 432 above) in injectable water, or in 2.5% injectable fructose aqueous solution, or in 5% normal saline; (b) adjusting the pH value to between 6 and 6.75; and sub-packaging the resultant solution in containers as unit-dose fluids; (c) placing the unit-dose fluids in a freeze-drier (lyophilizer); adjusting the temperature of the freeze drier to minus 35°C, and pumping the atmosphere of the freeze drier to below 40 Pa; (d) adjusting the temperature to between 3 and 5°C, and removing water completely; (e) adjusting the temperature of the freeze drier to 40-50°C and drying the obtained freeze-dried injectable powder; (f) blowing dry nitrogen into the freeze-drier, and sealing the bottles with sterile seal-capping; and (g) storing below 5°C in the dark

The amounts of β-lactam antibiotics and β-lactamase inhibitor were determined using C18 reverse-phase LC and UV-VIS detector (Tianjin Hemay BioTech Co., Ltd., analysis method No. HM-K-03). The content of aminoglycoside antibiotics was measured using reverse-phase HPLC and evaporation-light-scattering detector (ELSD detector) (Tianjin Hemay BioTech Co., Ltd., analysis method No. HM-K-08). The amount of antibiotic component at each time point was expressed as a percentage with respect to the amount at the initial time (herein defined as 100%). The relative amount of each antibiotic component in the composition at each time point was expressed as peak area ratio with respect to the corresponding peak area of an external standard.

### Example 1 (for comparative purposes)

Pharmaceutical composition of cefoperazone sodium and gentamicin.

Preparation

Cefoperazone sodium (4 g) was dissolved in 200 mL of injectable water, and gentamicin parenteral solution (80 mg in 2 mL of injectable water) was added. Upon mixing, a large amount of white solid precipitated out of the solution immediately.

### Example 2

Pharmaceutical composition of cefoperazone sodium, gentamicin, and buffer.

40 mg of cefoperazone sodium was dissolved in 2 mL of various buffer solutions with different pH value and concentration, and then 20 µL of gentamicin parenteral solution with concentration of 80 mg/2 mL was added. The mixture was ultrasonicated for 5 minutes and clarity observed. Clear solutions were obtained, and there was no precipitate generated when the pH value of buffer solution was above 6. The type of buffer solution used had little effect on the results. The results are shown in the table below.

| **Buffer solution** | **pH of buffer solutions** | **Concentration of buffer solutions (mM)** | **Results** |
|---|---|---|---|
| Citric acid/sodium citrate | 5 | 10 | *** |
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| Acetic acid/sodium acetate | 6.5 | 20 | OK |
| Phosphoric acid/ disodium hydrogen phosphate | 6.5 | 20 | OK |
| Arginine/arginine sodium | 6.5 | 20 | OK |
| *the solution turned slightly turbid, **the solution turned turbid, ***the solution turned fairly turbid, OK: clear solution | | | |

### Example 3

Pharmaceutical composition of cefoperazone sodium, sulbactam sodium, gentamicin, and buffer.

40 mg of cefoperazone sodium and 5 mg of sulbactam sodium were dissolved in 2 mL of various buffer solutions with different pH values and concentrations. Then, 20 µL of gentamicin parenteral solution having a concentration of 40 mg/mL was added. The mixture was ultrasonicated for 5 minutes and clarity observed. Clear solutions were obtained, and there was no precipitate generated when the pH value of buffer solution was above 6 and the concentration was higher than 20 mM. The type of buffer solution had little effect on the results. The results are shown in the table below.

| **Buffer solution** | **pH of buffer solution** | **Concentration of buffer solutions (mM)** | **Results** |
|---|---|---|---|
| Citric acid/sodium citrate | 5.5 | 10 | ** |
| Citric acid/sodium citrate | 6 | 10 | * |
| Citric acid/sodium citrate | 6.5 | 10 | OK |
| Citric acid/sodium citrate | 7.0 | 10 | OK |
| Citric acid/sodium citrate | 7.5 | 10 | OK |
| Citric acid/sodium citrate | 6 | 20 | OK |
| *the solution turned slightly turbid, **the solution turned turbid, | | | |
| ***the solution turned fairly turbid, OK: clear solution | | | |

### Example 4

Pharmaceutical composition of cefoperazone sodium, sulbactam sodium, gentamicin, sodium citrate, and citric acid.

Preparation

Sodium citrate (0.20 g) and citric acid (as much as necessary) were dissolved in 200 mL of injectable water to give a solution with the pH value of 6.75. Cefoperazone sodium (4 g) and sulbactam sodium (0.5 g) were then added, and the mixture was shaken. Gentamicin parenteral solution (80 mg in 2 mL) was added dropwise, and a small amount of white floc had precipitated out. After 15 min of ultrasonication, the white floc had dissolved to give a clear solution. The solution was packaged in a bottle or a bag and stored at room temperature. Observation of the appearance of the precipitate was conducted at 0, 1, 2, 4, and 6 hours. The amounts of cefoperazone sodium and sulbactam sodium were determined by sampling at the above-mentioned time points, and the results are shown below:

| **Time (h)** | **0** | **1** | **2** | **4** | **6** |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |

### Example 5

Pharmaceutical composition of cefoperazone sodium, sulbactam sodium, amikacin sulfate, and sodium citrate.

Preparation

Cefoperazone sodium (4 g), sulbactam sodium (0.5 g) and sodium citrate (0.20 g) were dissolved in 30 mL of injectable water; the pH value was adjusted to 6.75 with citric acid or sodium hydroxide aqueous solution. After 5 min of ultrasonication, a clear solution was obtained. The resultant solution was filtered (through a 0.2 µm filter), then loaded into containers and placed in a freeze-drier. The temperature of the freeze drier was adjusted to minus 35°C, and the vacuum of the freeze drier was pumped to below 30 Pa. Then the temperature was adjusted to 3°C, and water was removed completely. The resultant freeze-dried injectable powder was dried at 40°C. Dry nitrogen was blown in, and freeze-dried injectable powder of amikacin sulfate (500 mg) was added. The containers were sealed with sterile seal-caps, and stored in a refrigerator at 0°C. After 7 days, the freeze-dried injectable powder was dissolved in 100 mL of injectable water. Observation of the appearance of the precipitate was conducted at 0, 1, 2, 4, and 6 hours. The amounts of cefoperazone sodium, sulbactam sodium, and amikacin were determined by sampling at the above-mentioned time points, and the results are shown below:

| **Time (h)** | **0** | **1** | **2** | **4** | **6** |
|---|---|---|---|---|---|
| Cefoperazone sodium | 101.4 | 98.66 | 98.79 | 101.5 | 107.4 |
| Sulbactam sodium | 101.4 | 101.4 | 101.5 | 109.7 | 103.2 |
| Amikacin | 98.98 | 98.73 | 98.52 | 98.41 | 98.76 |

### Example 6

Pharmaceutical composition of cefoperazone sodium, sulbactam sodium, etimicin sulfate, and sodium citrate/citric acid.

Preparation

Cefoperazone sodium (4 g), sulbactam sodium (1 g) and sodium citrate (0.20 g) were dissolved in 30 mL of injectable water. The pH value was adjusted to 6.75 with citric acid or sodium hydroxide aqueous solution. 30 mL of the resultant solution was filtered (through a 0.2 µm filter) and then loaded into containers and placed into a freeze-drier. The temperature of the freeze drier was adjusted to minus 35°C, and the vacuum of the freeze drier was pumped to below 30 Pa. The temperature was adjusted to 3°C, and water was removed completely. The resultant freeze-dried injectable powder was dried at 40°C. Dry nitrogen was blown in, and freeze-dried injectable powder of etimicin sulfate (200 mg) was added and the containers were sealed with sterile seal-caps. The containers were stored in refrigerator at 0°C. After 7 days, the freeze-dried injectable powder was dissolved in 100 mL of injectable water. After 10 min of ultrasonication, a clear solution was obtained. Observation of the appearance of the precipitate was conducted at 0, 1, 2, 4, 6 and 8 hours. The amounts of cefoperazone sodium, sulbactam sodium, and etimicin were determined by sampling at the above-mentioned time points, and the results are shown below:

| **Time (h)** | **0** | **1** | **2** | **4** | **6** | **8** |
|---|---|---|---|---|---|---|
| Cefoperazone sodium | 110.4 | 99.48 | 101.3 | 98.66 | 98.02 | 99.30 |
| Sulbactam sodium | 99.62 | 100.4 | 100.2 | 100.2 | NA | NA |
| Etimicin | 108.9 | 100.2 | 100.5 | 100.4 | 100.4 | 97.50 |
| NA: undetermined content | | | | | | |

### Example 7

Pharmaceutical composition of ceftriaxone sodium, sulbactam sodium, gentamicin sulfate, sodium citrate, and citric acid.

Preparation

Citric acid and sodium citrate (0.20 g) were dissolved in 200 mL of injectable water to give a solution with the pH value of 6.75. Ceftriaxone sodium (4 g) and sulbactam sodium (1 g) were added, and the mixture was shaken. Gentamicin sulfate, 80 mg/2 mL parenteral solution, was added dropwise, and a small amount of white floc had formed. After 15 min of ultrasonication, the white floc had dissolved to give a clear solution. The solution was sealed and stored at room temperature (22°C). Observation of the appearance of the solution was conducted at 0, 1, 2, 4, 6 and 8 hours. The amounts of ceftriaxone sodium and sulbactam sodium were determined by sampling at the above-mentioned time points, and the results are shown below:

| **Time (h)** | **0** | **1** | **2** | **4** | **6** | **8** |
|---|---|---|---|---|---|---|
| Ceftriaxone sodium | 97.01 | 100.3 | 96.40 | 99.01 | 98.91 | 99.53 |
| Sulbactam sodium | 96.84 | 94.43 | 93.35 | 93.23 | 92.13 | 93.38 |

### Example 8

Pharmaceutical composition of ceftriaxone sodium, tazobactam sodium, gentamicin sulfate, sodium citrate, and citric acid.

Preparation

Sodium citrate (0.20 g) was dissolved in 200 mL of 2.5% aqueous fructose solution and citric acid was added in an amount sufficient to lower the pH value to 6.75. Ceftriaxone sodium (4 g) and tazobactam sodium (1 g) were added, and the mixture was shaken. Gentamicin sulfate parenteral solution (80 mg in 2 mL) was added dropwise, and a small amount of white floc has precipitated out. After 15 min of ultrasonication, the white floc had dissolved to give a clear solution.

100 mL of the resultant solution was filtered (through a 0.2 µm filter) into a container and placed into the freeze-drier. The freeze-drier temperature was adjusted to minus 35°C, and the atmosphere of the freeze-drier was pumped to 30 Pa. After removal of water at 3°C, the temperature of the freeze-drier was adjusted to 40°C to obtain freeze-dried injectable powder. Dry nitrogen was charged and the container was sealed with a sterile cover, and stored in a refrigerator at 0°C. After 7 days, the freeze-dried injectable powder was prepared as solution by mixing with 100 mL of injectable water. Observation of the appearance of precipitate was conducted at 0, 1, 2, 4, 6 and 8 hours. The amounts of ceftriaxone sodium and tazobactam sodium in the solution were determined, and the results are shown below:

| **Time (h)** | **0** | **1** | **2** | **4** | **6** | **8** |
|---|---|---|---|---|---|---|
| Ceftriaxone sodium | 98.75 | 100.1 | 99.97 | 98.87 | 99.27 | 100.5 |
| Tazobactam sodium | 102.2 | 97.53 | 94.16 | 94.58 | 95.45 | 94.44 |

### Example 9

Pharmaceutical composition of ceftriaxone sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Ceftriaxone sodium (4 g) were added, the mixture was then shaken. 100 mL of the resultant solution was filtered (though a 0.2µm filter) into a container and placed into a freeze-drier. The freeze-drier temperature was adjusted to minus 35°C and the atmosphere of the freeze-dried was pumped to 30 Pa. After removal of water at 3°C, the temperature of the freeze-drier was adjusted to 40°C to obtain freeze-dried injectable powder. Dry nitrogen was charged and the container was sealed with a sterile cover, and stored in a refrigerator at 0°C. After 7 days, the freeze-dried injectable powder was dissolved by mixing with 100 mL of 5% injectable fructose aqueous solution. Observation of the appearance of precipitate was conducted at 0, 1, 2, 4, 6 and 8 hours. The amount of ceftriaxone sodium in the solution were determined, and the results are shown below:

| **Time (h)** | **0** | **1** | **2** | **4** | **6** | **8** |
|---|---|---|---|---|---|---|
| Ceftriaxone sodium | 97.27 | 99.38 | 99.95 | 97.81 | 99.03 | 98.51 |

### Example 10

Pharmaceutical composition of mezlocillin sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Mezlocillin sodium (2 g) and sulbactam sodium (0.5 g) were added, and the mixture was then shaken. Etimicin sulfate (200 mg in 2 mL) was added with agitation, and small amount of white floc had precipitated out. After 15 min of ultrasonication, the white floc had dissolved to give a clear solution (22°C). The solution was sealed and stored at room temperature. Observation of the appearance of the precipitate was conducted at 0, 1, 2, 4, 6 and 8 hours. The amount of mezlocillin sodium, sulbactam sodium, and etimicin sulfate in the solution were determined, and the results are shown below

| **Time (h)** | **0** | **1** | **2** | **4** | **6** | **8** |
|---|---|---|---|---|---|---|
| Mezlocillin | 96.48 | 95.90 | 71.70 | 91.78 | 93.08 | 97.69 |
| Sulbactam sodium | 98.07 | 98.13 | 72.96 | 95.16 | 99.65 | 104.9 |
| Etimicin sulfate | 100.1 | 100.6 | 99.79 | NA | NA | NA |
| NA: undetermined content | | | | | | |

### Example 11

Pharmaceutical composition of mezlocillin sodium, sulbactam sodium, etimicin sulfate, sodium citrate, and citric acid.

Preparation

Sodium citrate (0.20 g) was dissolved in 200 mL injectable water and citric acid was added in an amount sufficient to lower the pH value to 6.75. Mezlocillin sodium (2 g) and sulbactam sodium (1 g) were added, the mixture was then shaken. 100 mL of the resultant solution was filtrated into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of 5% injectable fructose aqueous solution. 200 mg of etimicin sulfate was added and the mixture was agitated. A small amount of white floc had precipitated out. After 15 min of ultrasonication, the white floc had dissolved to give a clear solution. Observation of the appearance of the precipitate was conducted at 0, 1, 2, 4, 6 and 8 hours. The content of mezlocillin sodium, sulbactam sodium, and etimicin sulfate in the solution was determined, and the results are shown below:

| **Time (h)** | **0** | **1** | **2** | **4** | **6** | **8** |
|---|---|---|---|---|---|---|
| Mezlocillin sodium | 116.6 | 94.83 | 91.86 | 91.96 | 91.52 | 98.56 |
| Sulbactam sodium | 114.3 | 95.83 | 93.75 | 93.78 | 95.65 | 101.0 |
| Etimicin sulfate | 100.1 | 100.4 | 99.31 | 99.56 | 99.61 | 99.25 |

### Example 12

Pharmaceutical composition of mezlocillin sodium, etimicin sulfate, sodium citrate and citric acid.

Preparation

Sodium citrate (0.20 g) was dissolved in 200 mL injectable water and citric acid was added in an amount sufficient to lower the pH value to 6. Mezlocillin sodium (4 g) was added, the mixture was then shaken. 100 mL of the resultant solution was filtered (through a 0.2µm filter) into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C

After 7 days, the resultant freeze-dried injectable powder was reconstituted by mixing with 100 mL of 5% injectable fructose aqueous solution. 200 mg of etimicin sulfate was added and the mixture was agitated. A small amount of white floc had precipitated out. After 15 min of ultrasonication, the white floc had dissolved to give a clear solution. Observation of the appearance of the precipitate was conducted at 0, 1, 2, 4, 6 and 8 hours. The content of mezlocillin sodium in the solution was determined, and the results are shown below:

| **Time (h)** | **0** | **1** | **2** | **4** | **6** | **8** |
|---|---|---|---|---|---|---|
| Mezlocillin sodium | 95.05 | 96.63 | 89.33 | 98.72 | 92.73 | 92.51 |

### Example 13

Pharmaceutical composition of cefoperazone sodium, sulbactam sodium, gentamicin sulfate, sodium citrate, and citric acid.

Preparation

Cefoperazone sodium (4 g), sulbactam sodium (2 g), and sodium citrate (0.5 g) were dissolved in 10 mL of injectable water. The pH value of the solution was adjusted to 6.75 with citric acid. After 10 min of ultrasonication, a clear solution was obtained. 100 mL of the resultant solution was filtered (through a 0.2µm filter) into a container and placed into a freeze drier. The freeze-drier temperature was adjusted to minus 35°C, and the atmosphere of the freeze-dried was pumped to 30 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 16 mg of gentamicin sulfate. After 10 minutes of ultrasonication, a clear solution was obtained. Observation of the appearance of the precipitate was conducted at 0, 1, 2, 4, and 6 hours. The content of cefoperazone sodium and sulbactam sodium in the solution was determined, and the results are shown below:

| **Time (h)** | **0** | **1** | **2** | **4** | **6** |
|---|---|---|---|---|---|
| Cefoperazone sodium | 98.42 | 97.39 | 97.46 | 94.70* | 93.02* |
| Sulbactam sodium | 92.44 | 91.27 | 90.78 | 88.38* | 86.86* |
| *the solution turned slightly turbid | | | | | |

### Example 14

Pharmaceutical composition of cefoperazone sodium, sulbactam sodium, gentamicin sulfate, sodium citrate, and citric acid.

Preparation

Cefoperazone sodium (4 g), sulbactam sodium (4 g), and sodium citrate (0.20 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 30 Pa. After removal of water at 2°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 16 mg of gentamicin sulfate. After 10 minutes of ultrasonication, a clear solution was obtained. Observation of the appearance of the precipitate was conducted at 0, 1, 2, 4, and 6 hours. The content of cefoperazone sodium and sulbactam sodium in the solution was determined, and the results are shown below:

| **Time (h)** | **0** | **1** | **2** | **4** | **6** |
|---|---|---|---|---|---|
| Cefoperazone sodium | 101.7 | 96.06 | 95.72 | 97.32* | 69.95* |
| Sulbactam sodium | 99.50 | 96.28 | 102.5 | 95.78* | 70.74* |
| *the solution turned slightly turbid | | | | | |

### Example 15

Pharmaceutical composition of cefoperazone sodium, tazobactam sodium, etimicin sulfate, gentamicin sulfate, sodium citrate, and citric acid.

Preparation

Cefoperazone sodium (4 g), tazobactam sodium (1 g), and sodium citrate (0.2 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 40 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 20 mg of etimicin sulfate. After 10 minutes of ultrasonication, a clear solution was obtained. Observation of the appearance of the precipitate was conducted at 0, 1, 2, 4, 6 and 8 hours. The content of cefoperazone sodium and tazobactam sodium in the solution was determined, and the results are shown below:

| **Time (h)** | **0** | **1** | **2** | **4** | **6** | **8** |
|---|---|---|---|---|---|---|
| Cefoperazone sodium | 102.9 | 108.6 | 104.6 | 99.50 | 98.91 | 103.4 |
| Tazobactam sodium | 98.77 | 108.2 | 110.8 | 97.39 | 106.3 | 106.3 |

10 mL of the above final solution were separated and to it was added 16 mg of gentamicin sulfate. After 10 minutes of ultrasonication, a clear solution was obtained. Observation of the appearance of the precipitate was conducted at 0, 1, 2, 4, 6 and 8 hours. The content of cefoperazone sodium and tazobactam sodium in the solution was determined, and the results are shown below:

| **Time (h)** | **0** | **1** | **2** | **4** | **6** | **8** |
|---|---|---|---|---|---|---|
| Cefoperazone sodium | 92.44 | 104.6 | 102.4 | 97.95* | 98.90* | 102.9* |
| Tazobactam sodium | 93.36 | 104.8 | 100.0 | 96.58* | 106.1* | 107.1* |
| *the solution turned slightly turbid | | | | | | |

### Example 16

Pharmaceutical composition of cefoperazone sodium, sulbactam sodium, amikacin sulfate, sodium citrate, and citric acid.

Preparation

Cefoperazone sodium (4 g), sulbactam sodium (1 g), and sodium citrate (0.20 g) were dissolved in 10 mL injectable water. The pH value of the solution was adjusted to 6.75 with citric acid. After 10 min of ultrasonication, a clear solution was obtained. The resultant solution was filtered (though 0.2 µm filter) into a container and placed into a freeze-drier. The freeze-drier temperature was adjusted to minus 35°C, and the atmosphere of the freeze-dried was pumped to 40 Pa. After removal of water at 3°C, the temperature of the freeze-drier was adjusted to 40°C to obtain the freeze-dried injectable powder. Dry nitrogen was charged and the container was sealed with sterile cover, and stored in a refrigerator at 0°C.

After 7 days, the resultant freeze-dried injectable powder was reconstituted by mixing with 100 mL of water and allowed to stand at room temperature. 10 mL of this solution were separated and to it was added 40 mg amikacin sulfate. After 10 minutes of ultrasonication, a clear solution was obtained. Observation of the appearance of the precipitate was conducted at 0, 1, 2, 4, 6 and 8 hours. The content of cefoperazone sodium, sulbactam sodium and amikacin sulfate in the solution was determined, and the results are shown below:

| **Time (h)** | **0** | **1** | **2** | **4** | **6** | **8** |
|---|---|---|---|---|---|---|
| Cefoperazone sodium | 92.30 | 94.80 | 94.06 | 94.12 | 93.15 | 94.50 |
| Sulbactam sodium | 105.6 | 112.2 | 112.6 | 118.5 | 123.3 | 127.1 |
| Amikacin sulfate | 99.91 | 99.50 | 98.88 | 99.45 | 98.56 | 99.36 |

### Example 17

Pharmaceutical composition of cefoperazone sodium, sulbactam sodium, gentamicin sulfate, sodium citrate, and citric acid.

Preparation

Sodium citrate (0.20 g) was dissolved in 200 mL of injectable water. Enough citric acid was added to give a solution having the pH value of 6.75. Ceftriaxone sodium (2 g) and sulbactam sodium (2 g) were added, and the mixture was shaken. Gentamicin sulfate (160 mg) was added and the mixture was agitated. A small amount of white floc had formed. After 15 minutes of ultrasonication, a clear solution was obtained. The solution was sealed and stored at room temperature. Observation of the appearance of the precipitate was conducted at 0, 1, 2, 4, 6 and 8 hours. The content of ceftriaxone sodium and sulbactam sodium in the solution was determined, and the results are shown below:

| **Time (h)** | **0** | **1** | **2** | **4** | **6** | **8** |
|---|---|---|---|---|---|---|
| Ceftriaxone sodium | 97.01 | 100.3 | 96.40 | 99.01 | 98.91 | 99.53 |
| Sulbactam sodium | 96.84 | 94.43 | 93.35 | 93.23 | 92.13 | 93.38 |

### Example 18

Pharmaceutical composition of ceftriaxone sodium, tazobactam sodium, gentamicin sulfate, sodium citrate, and citric acid.

Preparation

Sodium citrate (0.20 g) was dissolved in 200 mL of injectable water. Enough citric acid was added to give a solution having the pH value of 6.75. Ceftriaxone sodium (2 g) and tazobactam sodium (2 g) were added, and the mixture was shaken. Gentamicin sulfate (160 mg) was added and the mixture was agitated. A small amount of white floc had formed. After 15 minutes of ultrasonication, a clear solution was obtained. The solution was sealed and stored at room temperature. Observation of the appearance of the precipitate was conducted at 0, 1, 2, 4, 6 and 8 hours. The content of ceftriaxone sodium and tazobactam sodium in the solution was determined, and the results are shown below

| **Time (h)** | **0** | **1** | **2** | **4** | **6** | **8** |
|---|---|---|---|---|---|---|
| Ceftriaxone sodium | 98.75 | 100.1 | 99.97 | 98.87 | 99.27 | 100.5 |
| Tazobactam sodium | 102.2 | 97.53 | 94.16 | 94.58 | 95.45 | 94.44 |

## Claims

1. A pharmaceutical composition, comprising:
at least one β-lactam antibiotic selected from cefoperazone, ceftriaxone, mezlocillin, or a pharmaceutically acceptable salt or hydrate thereof;
at least one aminoglycoside antibiotic selected from amikacin, gentamicin, or etimicin, or a pharmaceutically acceptable salt or hydrate thereof; and
at least one buffer component having an effective pH range of between 5.5 and 7.5, for use as an anti-microbial and anti-infection drug.

2. The pharmaceutical composition of claim 1, wherein said buffer component is citric acid/citrate.

3. The pharmaceutical composition of claim 1, wherein an effective pH range of said buffer component is between 6 and 6.75.

4. The pharmaceutical composition of claim 1, further comprising at least one β-lactamase inhibitor.

5. The pharmaceutical composition of claim 4, wherein said β-lactamase inhibitor is selected from clavulanic acid, sulbactam, tazobactam, or a pharmaceutically acceptable salt or a hydrate thereof.

6. The pharmaceutical composition of claim 1 or 4, provided in a unit dose Formulation, said unit dose comprising between 0.01 g and 5 g of said aminoglycoside antibiotic.

7. The pharmaceutical composition of claim 1 or 4, provided in a unit dose Formulation, said unit dose comprising between 0.1 g and 5 g of said β-lactam antibiotic.

8. The pharmaceutical composition of claim 4, provided in a unit dose Formulation, said unit dose comprising between 0.1 g and 5 g of said β-lactamase inhibitor.

9. The pharmaceutical composition of claim 1 or 4, comprising further at least one iso-osmotic component.

10. The pharmaceutical composition of claim 9, wherein said iso-osmotic component is glucose, fructose, or sodium chloride.

11. The pharmaceutical composition of claim 1, 4 or 9 provided as a parenteral solution, powder for injection, or lyophilized powder for injection.

12. A method for preparing the pharmaceutical composition of claim 11 in a form of lyophilized powder for injection, the method comprising the steps of:
(a) dissolving a β-lactam antibiotic, a buffer component, an aminoglycoside antibiotic, and other components of the pharmaceutical composition in injectable water, in 2.5% injectable fructose aqueous solution, or in 5% normal saline; adjusting the pH value to between 6 and 6.75 with citric acid/sodium citrate; and obtaining a clear solution after stirring;
(b) dividing the clear solution obtained in step (a) into unit doses, each unit dose being placed in an individual container; placing the containers in a freeze-drier precooled to minus 5°C; and adjusting the temperature of the freeze drier to about minus 35°C;
(c) evacuating the atmosphere in the freeze drier to below 40 Pa;
(d) adjusting the temperature in the freeze drier to between 3 and 5°C;
(e) removing water completely under the above-mentioned conditions;
(f) adjusting the temperature of the freeze drier to between 40 and 50°C, and drying freeze-dried injectable powder obtained in step (e);
(g) charging nitrogen into the freeze drier, sealing the containers with sterile seal-caps, and storing the containers below 5°C in the dark.

## Patentansprüche

1. Pharmazeutische Zusammensetzung umfassend:
mindestens ein β-Lactam-Antibiotikum ausgewählt unter Cefoperazon, Ceftriaxon, Mezlocillin oder einem pharmazeutisch akzeptablen Salz oder Hydrat davon;
mindestens ein Aminoglykosid-Antibiotikum ausgewählt unter Amikacin, Gentamicin oder Etimicin oder einem pharmazeutisch akzeptablen Salz oder Hydrat davon; und
mindestens eine Pufferkomponente, die einen wirksamen pH-Wertbereich zwischen 5,5 und 7,5 aufweist, zur Verwendung als antimikrobielles oder Antiinfektionsmedikament.

2. Pharmazeutische Zusammensetzung nach Anspruch 1, wobei die Pufferkomponente Zitronensäure/Zitrat ist.

3. Pharmazeutische Zusammensetzung nach Anspruch 1, wobei ein wirksamer pH-Wertbereich der Pufferkomponent zwischen 6 und 6,75 liegt.

4. Pharmazeutische Zusammensetzung nach Anspruch 1, des Weiteren mindestens eine β-Lactamaseinhibitor umfassend.

5. Pharmazeutische Zusammensetzung nach Anspruch 4, wobei der β-Lactamaseinhibitor unter Clavulansäure, Sulbactam, Tazobactam oder einem pharmazeutisch akzeptablen Salz oder einem Hydrat davon ausgewählt wird.

6. Pharmazeutische Zusammensetzung nach Anspruch 1 oder 4, in einer Einheitsdosisrezeptur bereitgestellt, wobei die Einheitsdosis 0,01 g bis 5 g des Aminoglykosid-Antibiotikums umfasst.

7. Pharmazeutische Zusammensetzung nach Anspruch 1 oder 4, in einer Einheitsdosisrezeptur bereitgestellt, wobei die Einheitsdosis 0,1 g bis 5 g des β-Lactam-Antibiotikums umfasst.

8. Pharmazeutische Zusammensetzung nach Anspruch 4, in einer Einheitsdosisrezeptur bereitgestellt, wobei die Einheitsdosis 0,1 g bis 5 g des β-Lactamaseinhibitors umfasst.

9. Pharmazeutische Zusammensetzung nach Anspruch 1 oder 4, die des Weiteren mindestens eine isoosmotische Komponente umfasst.

10. Pharmazeutische Zusammensetzung nach Anspruch 9, wobei die isoosmotische Komponente Glucose, Fruktose oder Natriumchlorid ist.

11. Pharmazeutische Zusammensetzung nach Anspruch 1, 4 oder 9, als parenterale Lösung, Pulver zur Injektion oder lyophilisiertes Pulver zur Injektion bereitgestellt.

12. Verfahren zur Herstellung der pharmazeutischen Zusammensetzung nach Anspruch 11 in Form von lyophilisiertem Pulver zur Injektion, wobei das Verfahren die Schritte umfasst des:
(a) Lösens eines β-Lactam-Antibiotikums, einer Pufferkomponente, eines Aminoglykosid-Antibiotikums und anderer Komponenten der pharmazeutischen Zusammensetzung in injizierbarem Wasser, in 2,5%iger injizierbarer wässriger Fruktoselösung oder in 5%iger normaler physiologischer Kochsalzlösung; Einstellens des pH-Werts mit Zitronensäure/Natriumcitrat auf 6 bis 6,75; und Erhaltens einer klaren Lösung nach Rühren;
(b) Teilens der in Schritt (a) erhaltenen klaren Lösung in Einheitsdosen, wobei jede Einheitsdosis in einen einzelnen Behälter gegeben wird; Eingebens der Behälter in einen Gefriertrockner, der auf minus 5°C vorgekühlt worden ist; und Einstellens der Temperatur des Gefriertrockners auf etwa minus 35°C;
(c) Auspumpens der Atmosphäre in dem Gefriertrockner auf unter 40 Pa;
(d) Einstellens der Temperatur in dem Gefriertrockner auf 3 bis 5°C;
(e) vollständigen Entfernens von Wasser unter den oben erwähnten Bedingungen;
(f) Einstellens der te1 des Gefriertrockners auf 40 bis 50°C und Trocknens des in Schritt (e) erhaltenen, gefriergetrockneten, injizierbaren Pulvers.
(g) Einführens von Stickstoff in den Gefriertrockner, dicht Verschließens der Behälter mit sterilen Siegelkappen und Lagerns der Behälter bei unter 5°C im Dunkeln.

## Revendications

1. Composition pharmaceutique, comprenant :
au moins un antibiotique β-lactame sélectionné parmi la céfopérazone, la ceftriaxone, la mezlocilline, ou un sel pharmaceutiquement acceptable ou un hydrate de ceux-ci ;
au moins un antibiotique aminoglycosidique sélectionné parmi l'amikacine, la gentamicine, ou l'étimicine, ou un sel pharmaceutiquement acceptable ou un hydrate de ceux-ci ; et
au moins un composant tampon ayant une plage de pH efficace comprise entre 5,5 et 7,5, pour une utilisation en tant que médicament antimicrobien et anti-infectieux.

2. Composition pharmaceutique selon la revendication 1, dans laquelle ledit composant tampon est un mélange d'acide citrique/citrate.

3. Composition pharmaceutique selon la revendication 1, dans laquelle une plage de pH efficace dudit composant tampon est comprise entre 6 et 6,75.

4. Composition pharmaceutique selon la revendication 1, comprenant en outre au moins un inhibiteur de la β-lactamase.

5. Composition pharmaceutique selon la revendication 4, dans laquelle ledit inhibiteur de la β-lactamase est sélectionné parmi l'acide clavulanique, le sulbactame, le tazobactame ou un sel pharmaceutiquement acceptable ou un hydrate de ceux-ci.

6. Composition pharmaceutique selon la revendication 1 ou 4, proposée dans une formulation de dose unitaire, ladite dose unitaire comprenant entre 0,01 g et 5 g dudit antibiotique aminoglycosidique.

7. Composition pharmaceutique selon la revendication 1 ou 4, proposée dans une formulation de dose unitaire, ladite dose unitaire comprenant entre 0,1 g et 5 g dudit antibiotique β-lactame.

8. Composition pharmaceutique selon la revendication 4, proposée dans une formulation de dose unitaire, ladite dose unitaire comprenant entre 0,1 g et 5 g dudit inhibiteur de la β-lactamase.

9. Composition pharmaceutique selon la revendication 1 ou 4, comprenant en outre au moins un composant iso-osmotique.

10. Composition pharmaceutique selon la revendication 9, dans laquelle ledit composant iso-osmotique est le glucose, le fructose, ou le chlorure de sodium.

11. Composition pharmaceutique selon la revendication 1, 4 ou 9, proposée sous forme de solution parentérale, poudre pour injection, ou poudre lyophilisée pour injection.

12. Procédé de préparation de la composition pharmaceutique selon la revendication 11 sous forme de poudre lyophilisée pour injection, le procédé comprenant les étapes consistant à :
(a) dissoudre un antibiotique β-lactame, un composant tampon, un antibiotique aminoglycosidique, et d'autres composants de la composition pharmaceutique dans de l'eau injectable, dans une solution aqueuse de 2,5 % de fructose injectable, ou dans une solution saline normale à 5 % ; ajuster la valeur de pH entre 6 et 6,75 avec un mélange d'acide citrique/citrate de sodium ; et obtenir une solution limpide après agitation ;
(b) diviser la solution limpide obtenue dans l'étape (a) en doses unitaires, chaque dose unitaire étant placée dans un contenant individuel ; placer les contenants dans un lyophilisateur préalablement refroidi à - 5 °C ; et ajuster la température du lyophilisateur à environ -35 °C ;
(c) évacuer l'atmosphère dans le lyophilisateur à moins de 40 Pa ;
(d) ajuster la température dans le lyophilisateur entre 3 et 5 °C ;
(e) éliminer complètement l'eau dans les conditions mentionnées ci-dessus ;
(f) ajuster la température du lyophilisateur entre 40 et 50 °C, et sécher la poudre lyophilisée injectable obtenue dans l'étape (e) ;
(g) charger de l'azote dans le lyophilisateur, sceller les contenants avec des bouchons d'étanchéité stériles, et stocker les récipients à moins de 5 °C dans l'obscurité.
